(19) Europäisches Patentamt
European Patent Office
Office européen des brevets

(11) Publication number: **0 003 084**
**B1**

(12) **EUROPEAN PATENT SPECIFICATION**

(45) Date of publication of patent specification: **24.02.82**

(21) Application number: **78300912.9**

(22) Date of filing: **27.12.78**

(51) Int. Cl.$^3$: **C 07 D 307/94,**
C 07 D 405/04,
C 07 D 413/04,
A 61 K 31/34 //C07D307/32,
C07D493/10

(54) **Spirobenzofuranone compounds, processes for their preparation and their use as medicines.**

(30) Priority: **27.12.77 JP 159177/77**
**19.06.78 JP 74700/78**
**06.11.78 JP 136967/78**

(43) Date of publication of application:
**25.07.79 Bulletin 79/15**

(45) Publication of the grant of the patent:
**24.02.82 Bulletin 82/8**

(84) Designated Contracting States:
**BE CH DE FR GB IT NL SE**

(56) References cited:
**CHEMICAL ABSTRACTS, vol. 88, no. 7, February 13, 1978, page 520, ref. 50577f Columbus, Ohio, USA**
**M. OKITSU et al.: "A novel ring contraction of 4-bromo-2,3-dihydrobenzo(b)thiepin-5(4H)-one" CHEMISTRY AND INDUSTRY, August 19, 1967, London**
**J. A. DONNELY et al.: "Reactions methylsulphoxonium methylide" pages 1402, 1403.**

(73) Proprietor: **Takeda Chemical Industries, Ltd.**
**27, Doshomachi 2-Chome**
**Higashi-ku Osaka, 541 (JP)**

(72) Inventor: **Hirosada, Sugihara**
**5-604, 2 Minase 2-chome Shimamotocho**
**Mishima-gun**
**OSAKA 618 (JP)**
Inventor: **Watanable, Masazumi**
**4-54, Seiwadainishi 2-chome Kawanishi**
**Hyogo 666-01 (JP)**
Inventor: **Mitsuru, Kawada**
**12-203, 14 Mukonosohonmachi 3-chome**
**Amagasaki**
**Hyogo 661 (JP)**
Inventor: **Isuke, Imada**
**18-10, Tsuruyamadai 4-chome Isumi**
**Osaka 594 (JP)**

(74) Representative: **Laredo, Jack Joseph et al,**
**Elkington and Fife High Holborn House 52/54**
**High Holborn**
**London, WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 003 084 B1

Spirobenzofuranone compounds, processes for their preparation and their use as medicines

This invention relates to spiro compounds having a novel skeletal structure, which are of use as medicines and as intermediates for the production of medicines. The invention also relates to methods of producing these novel spiro compounds.

J. A. Donnelly *et al.* (Chemistry and Industry, August 19, 1967, pages 1402—3) describe in their communication entitled: "Reactions of Chromindogenides with Dimethylsulphoxonium Methylide", the use of the latter reagents (DMSOY) to form spirocyclopropyl ketones from flavanoid structures containing exocyclic double bonds. Thus, chromindogenide, flavindogenide and aurone were converted to the corresponding spirocyclopropyl ketones upon reaction with DMSOY.

The present invention relates to novel spiro compounds of the formula:

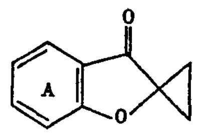

(I)

wherein Ring A represents a benzene ring or a naphthalene ring, the ring being unsubstituted or substituted by at least one of $C_{1-6}$ alkyl, nitro, halogen, amino, which may optionally itself be substituted, hydroxyl which may optionally itself be substituted, acyl and sulphamoyl,

and to methods of producing these novel spiro compounds.

The optional substituents of Ring A (as defined above) are now described in detail. Examples of the $C_{1-6}$-alkyl group include, e.g. methyl, ethyl, *n*-propyl, iso-propyl, *n*-butyl, iso-butyl, sec.-butyl, tert.-butyl, *n*-pentyl, iso-pentyl, *n*-hexyl and 2-methylphenyl or 2-ethylbutyl. The halogen may be chlorine, bromine, fluorine or iodine.

Examples of the amino group, which may optionally be substituted, include amino, mono- or di-alkylamino, acylamino, sulphonylamino and cycloamino. The mono- or dialkylamino group may be amino which is mono- or di-substituted by alkyl groups of 1 to 4 carbon atoms, such as e.g., methylamino, ethylamino, *n*-propylamino, iso-propylamino, *n*-butylamino, dimethylamino, diethylamino, di-*n*-propylamino or methylethylamino. The acylamino group may, for example, be alkanoylamino containing 2 to 4 carbon atoms (e.g. acetylamino, propionylamino, *n*-butyrylamino or iso-butyrylamino). The sulphonylamino group may, for example, be alkanesulphonylamino containing 1 to 4 carbon atoms (e.g. methanesulphonylamino or ethanesulphonylamino). As the cycloamino group, there may be mentioned 5- or 6-membered cycloamino groups which may contain N or O, for example, pyrrolidinyl, piperidino, piperazinyl or morpholino. The piperazinyl group may have a substituent at the nitrogen atom of its 4-position, such as an alkyl group containing 1 to 4 carbon atoms (e.g. methyl or ethyl), a phenyl-$C_{1-4}$ alkyl group (e.g. benzyl) or an alkanoyl group containing 2 to 4 carbon atoms (e.g. acetyl or propionyl).

As examples of the hydroxyl group which may optionally be substituted, there may be mentioned hydroxyl, alkoxy, aralkyloxy or acyloxy. The alkoxy group preferably contains 1 to 6 carbon atoms (e.g. methoxy, ethoxy, *n*-propoxy, iso-propoxy, *n*-butoxy, iso-butoxy, sec.-butoxy- or tert.-butoxy), and the alkoxy group may be further substituted, for example, by mono- or di-$C_{1-4}$ alkylamino groups (e.g. methylamino, ethylamino, dimethylamino or diethylamino). The aralkyloxy group may, for example, be a phenyl-$C_{1-4}$ alkyloxy group (e.g. benzyloxy or phenethyloxy). The acyloxy group is preferably an alkanoyloxy group containing 2 to 6 carbon atoms (e.g. acetyloxy, propionyloxy, *n*-butyryloxy or iso-butyryloxy), or benzoyloxy group, for instance.

The acyl groups may, for example, be an alkanoyl group of 2 to 6 carbon atoms (e.g. acetyl, propionyl, *n*-butyryl or iso-butyryl) or benzoyl.

The substituents of Ring A (up to 4 at a maximum) may be present in various substitutable positions on Ring A, and may be the same or different. When A is benzene, the A Ring is preferably substituted at its 5- or 6-position (the 5-position is the more desirable), by an amino group which may optionally be substituted (especially a mono- or di-$C_{1-4}$ alkylamino group), or by an acyl group (especially a $C_{2-4}$ alkanoyl group).

The spiro compound (I) of the present invention may be produced, for instance by, decarboxylating a compound of the formula:

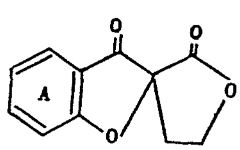

(II)

wherein Ring A is as defined hereinbefore.

This reaction is normally carried out in the presence of a catalyst which assists in the decarboxylation. Among preferred catalysts for this purpose are metal halides (e.g. sodium chloride, sodium bromide, sodium iodide, potassium bromide, potassium chloride or potassium iodide), and quaternary ammonium salts (e.g. tetramethyl-ammonium bromide). The reaction temperature is normally from 100°C to 200°C, and preferably from 140°C to 160°C, although the reaction may be conducted at higher or lower temperatures if it is desired to control the reaction velocity. Purging the reaction vessel with an inert gas (e.g. $N_2$ or argon) is sometimes effective in preventing side-reactions and improving yields. This reaction is normally carried out in a suitable solvent. While any solvent that will not interfere with the reaction may be employed, it is normally advantageous to employ a solvent having a boiling point higher than the reaction temperature (e.g. dimethyl sulphoxide, N,N-dimethylformamide or hexamethylphosphoramide).

Among the spiro compound (I) of this invention, those having a substituent or substituents on Ring A can also be produced by subjecting a compound (I) wherein Ring A is unsubstituted, or a compound (I) having at least one hydrogen atom on its Ring A, to a *per se* conventional alkylation, nitration, halogenation or acylation, depending on the kinds of the then intended substituents. A compound (I) wherein the substituent(s) on Ring A is (are) an amino group(s) can be produced also by subjecting a compound (I) wherein the position(s) to which an amino group(s) is (are) to be introduced is (are) occupied by a hydrogen atom(s), to a nitration reaction and, then, to a reduction reaction such as catalytic reduction reaction.

Further, it is also possible to replace the substituent(s) on Ring A of a compound (I) with other substituent(s) by reactions known *per se*. Thus, a compound (I) wherein the substituent(s) is (are) mono- or di-alkylamino group(s) can be produced by, for example, subjecting a compound (I) wherein the substituent(s) is (are) an amino group(s) to reductive alkylation, i.e., to reduction with a metal hydride such as sodium cyanoborohydride, or to catalytic reduction in the presence of a carbonyl compound (e.g. formalin, acetaldehyde or acetone), or to a reaction with an alkyl halide to cause mono- or dialkylation. A compound (I) having mono- or dialkylamino group(s) can also be produced by subjecting a compound (I) wherein the substituent(s) is (are) a nitro group(s), to catalytic reduction with a catalyst such as platinum oxide or Raney nickel in the presence of the above-mentioned carbonyl compound.

The above-mentioned production of the compound (I) having a mono- or di-alkylamino substituent(s) may, for example, be illustrted by the following reaction scheme:

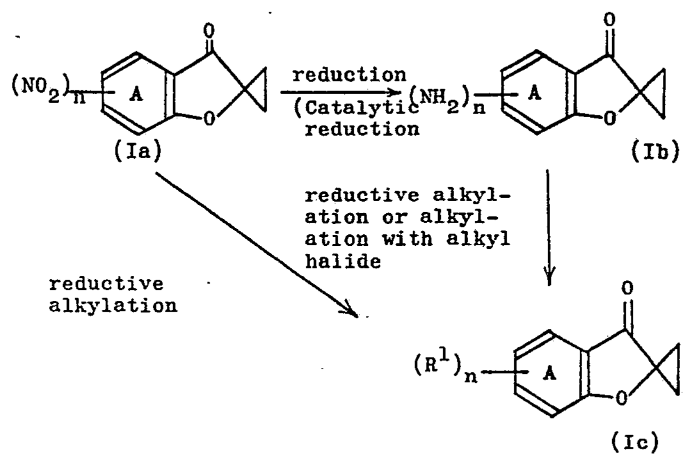

(Ia) (Ib) (Ic)

(Ib)

[wherein ring A is as defined above, *n* is 1 or 2 and $R^1$ represents mono- or di-alkyl amino as defined hereinbefore.]

The contemplated compound (I) obtained in the foregoing manner can be isolated from the reaction mixture and purified by conventional procedures (e.g. distillation, recrystallization or column chromatography). According to the types of substituents on Ring A, the compound (I) may be isolated as pharmaceutically acceptable salts. For example, when an amino group (e.g. amino, mono- or di-alkylamino or cycloamino) is present as the substituent, the compound (I) can be isolated as an acid addition salt (e.g. a mineral acid salt such as the hydrochloride or hydrobromide, or an organic acid salt such as the citrate or oxalate), or when the substituent is a hydroxyl group, the compound (I) can be isolated as an alkali metal salt (e.g. the sodium salt or potassium salt.

The spiro compounds (I) according to this invention are compounds having a novel skeletal structure, which exhibit gastric secretion inhibitive, antiinflammatory, analgesic and other actions in mammalian animals (e.g. man, rat, mouse, guinea pig), for instance, and are of value as anti-ulcer, antiinflammatory, analgesic and other drugs for the management of peptic ulcers, acute or chronic gastritis, lumbago, arthritis and other diseases. in such medicinal applications, each compound (I) can be safely administered orally or parenterally, either as it is or as formulated with pharmaceutically acceptable

carriers or diluents known *per se* into suitable dosage forms such as tablets, powders, capsules, injections and suppositories. While the recommended dosage depends on the subject, the condition, the route of administration, etc., the normal oral dosage for the treatment of peptic ulcers or acute or chronic gastritis is 1 mg. to 20 mg. as compound (I) per kg body weight per dose, to be given from once to 3 times daily.

The starting compound (II) which is employed in the practice of this invention can be prepared by the following synthesis route or by any process analogous thereto.

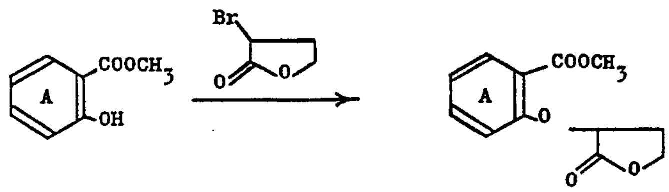

$(CH_3CO)_2O$, $(CH_2H_5)_3N$ or

1) Hydrolysis, lactonization
2) $(CH_3CO)_2O$, $(C_2H_5)_3N$

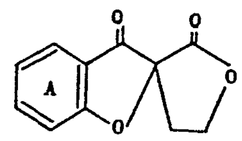

(wherein Ring A is as defined hereinbefore)

The following pharmacological test, and the following reference and working examples are intended to describe this invention in further detail but should not be considered to limit the scope of this invention in any way.

*Pharmacological Test*

The pharmacological activity of the compounds (I) of this invention was assayed by a gastric-juice-secretion-inhibition test with rats, the results of which are as follows.

In accordance with the method described in "Gastroenterology" *5*, 43 (1945), the inhibition of gastric-juice-secretion was evaluated by means of pylorus ligated rats.

Five each of male Sprague-Dawley rats (each weighing 100-130 g.) were used for the control and five test groups. Each animal eas deprived of food for 18 hours before the test, except for drinking water. The pylorus of each animal was ligated under anaesthesia with ether, and each test compound was then intraduodenally administered to the animals of each test group at a dosage of 50 mg./kg Three hours after the ligation, the animals were sacrificed. The gastric secretions of the tested animals were collected and subjected to centrifuging for 10 minutes (3,500 r.p.m.), and the volume of gastric juice was measured. The results are shown in the table below.

The compounds were administered orally to ICR-type mice in groups of five animals at a dosage of 500 mg./kg. so as to examine acute toxicity. No mouse was dead during 7 days in any group.

TABLE

Inhibition of Gastric-Juice Secretion in Rats

| Compound 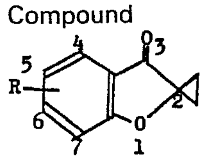 | Dose (i.d.mg./kg.) | Inhibition of Secretion (%) |
|---|---|---|
| 5-Cl | 50 | 48 |
| 5-$NO_2$ | 50 | 57 |
| 5-$N(CH_3)_2$ | 50 | 78 |
| 4-Br, 5-$NH_2$ | 50 | 53 |
| 5-$NHSO_2CH_3$ | 50 | 68 |

Reference Example 1

25 g. of α-bromo-γ-butyrolactone were added dropwise under ice-cooling to a mixture of 15.2 g. of methyl salicylate, 12 g. of sodium hydroxide and 150 ml. of N,N-dimethylformamide. The resulting mixture was stirred at room temperature for 28 hours. The reaction mixture was made acidic by the addition of dilute hydrochloric acid and extracted with ethyl acetate. The extract was washed with water, dried and concentrated under reduced pressure. The residue was dissolved in 30 ml. of methanol; 150 ml. of a 20% aqueous solution of sodium hydroxide were then added dropwise and the solution was stirred at 55°C for 30 minutes. The reaction mixture was made acidic with 60 ml. of concentrated hydrochloric acid, the resulting precipitate (salicylic acid) was filtered off and the filtrate was extracted with ethyl acetate. The extract was washed with water, dried and concentrated under reduced pressure.

The residue was dried *in vacuo* over phosphorus pentoxide for 24 hours, after which it was recrystallized from ethyl acetate-*n*-hexane (2:1). By the above procedure, there were obtained 8.0 g. of α-[(2-carboxyphenyl)oxy]-γ-butyrolactone as colourless needles melting at 113—115°C. (as determined by the Hot-Plate method; in all the examples hereinafter, the same method was applied to the determination of melting points).

Elemental analysis, for $C_{11}H_{10}O_5$

| | | |
|---|---|---|
| Calcd.: | C, 59.46; | H, 4.54 |
| Found: | C, 59.21; | H, 4.51 |

Reference Example 2

Using 18.7 g of methyl 5-chlorosalicylate, the procedure of Reference Example 1 was repeated to obtain 9.3 g of α-[(2-carboxy-4-chlorophenyl)oxy]-γ-butyrolactone as colourless needles melting at 159—160.5°C.

Elemental analysis, for $C_{11}H_9ClO_5$

| | | | |
|---|---|---|---|
| Calcd.: | C, 51.48; | H, 3.53; | Cl, 13.82 |
| Found: | C, 51.22; | H, 3.50; | Cl, 13.70 |

Reference Example 3

To a solution of 16.6 g of methyl 3-methylsalicylate in 200 ml of dimethylformamide were added 5.3 g of sodium hydride (50% suspension in Bayol 85 trade mark).

Then, under ice-cooling, a solution of 18.2 g of α-bromol-γ-butyrolactone in 10 ml of dimethylformamide was added dropwise. The mixture was stirred at room temperature for 10 hours, after which time it was diluted with a small amount of water and distilled under reduced pressure to remove the solvent.

60 ml of a 20% aqueous solution of sodium hydroxide was added to the residue and the resulting mixture was stirred at 50—60°C for one hour. The reaction mixture was made acidic with 40 ml of concentrated hydrochloric acid, and the precipitated crystals were collected by filtration to recover the unreacted 3-methylsalicylic acid. The filtrate was extracted with ethyl acetate, washed with water, dried and distilled under reduced pressure to remove the solvent. The residue was dried over phosphorus pentoxide at 50°C for 12 hours, after which it was recrystallized from ethyl acetate-hexane. By the above procedure there were obtained 12 g of α-[(2-carboxy-6-methylphenyl)oxy]-γ-butyrolactone as colourless needles melting at 129—131°C.

Elemental analysis, for $C_{12}H_{12}O_5$

| | | |
|---|---|---|
| Calcd.: | C, 61.01; | H, 5.12 |
| Found: | C, 61.00; | H, 5.12 |

Reference Example 4

Using 22 g of methyl 3,5-dichlorosalicylate, the reaction procedure of Reference Example 3 was repeated to yield 14 g of α-[(2-carboxy-4,6-dichlorophenyl)oxy]-γ-butyrolactone as colourless crystals melting at 117—120°C.

Elemental analysis, for $C_{11}H_8Cl_2O_5$

| | | |
|---|---|---|
| Calcd.: | C, 45.38; | H, 2.77 |
| Found: | C, 45.43; | H, 2.66 |

Reference Example 5

30.7 g of α-bromo-γ-butyrolactone were added under cooling with ice to a mixture of 32 g of

methyl 5-benzyloxysalicylate, 17 g of anhydrous potassium carbonate and 500 ml of acetone and the resulting mixture was refluxed for 15 hours. After cooling, the acetone was distilled off and 10% methanolic sodium hydroxide was added to the residue to achieve hydrolysis. The reaction mixture was made acidic with hydrochloric acid and extracted with ethy acetate. The extract was washed with water, dried over anhydrous sodium sulphate and distilled to remove the solvent. The residue was dissolved in dioxane (300 ml)-benzene (200 ml), and the resulting solution was refluxed in the presence of *p*-toluene-sulphonic acid (30 g), with the resulting water being continuously distilled off. The solvent was distilled off and the residue was diluted with water and extracted with ethyl acetate. The extract was washed with water, dried and concentrated to remove the solvent. The residue was recrystallized from ethyl acetate. By the above procedure, there was obtained $\alpha$-[(2-carboxy-4-benzyloxyphenyl)oxy]-$\gamma$-butyrolactone as colourless needles, m.p. 120—122°C.
Yield: 21.5 g.

Elemental analysis, for $C_{18}H_{16}O_6$

Calcd.: C, 65,85; H, 4.91
Found: C, 65.86; H, 4.96

Reference Example 6—12

The following compounds were produced by a procedure similar to that described in Reference Example 5.

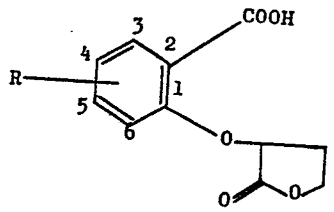

| Reference Example | Compound R | m.p. (°C) | Molecular formula | Elemental Analysis (Upper rank: Calcd. / Lower rank: Found.) C | H |
|---|---|---|---|---|---|
| 6 | 5-$OCH_3$ | 130–133 | $C_{12}H_{12}O_6$ | 57.14<br>57.08 | 4.80<br>4.75 |
| 7 | 4-$OCH_3$ | 129–132 | $C_{12}H_{12}O_6$ | 57.14<br>57.04 | 4.80<br>4.78 |
| 8 | 4-$COCH_3$ | 155–158 | $C_{13}H_{12}O_6$ | 59.09<br>58.98 | 4.58<br>4.48 |
| 9 | 3-OH | 189–198 (decomp.) | $C_{11}H_{10}O_6$ | 55.46<br>55.51 | 4.23<br>4.10 |
| 10 | 4,5- 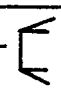 | 183–187 (decomp.) | $C_{15}H_{12}O_5$ | 66.17<br>66.06 | 4.44<br>4.22 |
| 11 | 4-$C_6H_{13}$ | 98–100 | $C_{17}H_{22}O_5$ | 66.65<br>66.50 | 7.24<br>7.28 |
| 12 | 5-$CH(CH_3)_2$ | 124–126 | $C_{14}H_{16}O_5$ | 63.62<br>63.60 | 6.10<br>6.18 |

Reference Example 13

51 g. of Methyl 4-acetylamino-5-chloro-2-hydroxybenzoate and 36.8 g. of anhydrous potassium carbonate were suspended in 350 ml. of N,N-dimethylformamide. 55 g. of $\alpha$-bromo-$\gamma$-butyrolactone were added to the suspension, and the resulting mixture was stirred at 60°C for 12 hours. The solvent was evaporated off under reduced pressure. The residue was diluted with water and extracted with ethyl acetate. The extract was washed with water, dried and concentrated to remove the solvent. The residue was dissolved in chloroform, and subjected to column chromatography on silica gel, using

chloroform as the eluent. The product was recrystallized from methanol. By the above procedure, there was obtained $\alpha$-[(5-acetylamino-4-chloro-2-methoxycarbonylphenyl)oxy]-$\gamma$-butyrolactone as pale yellow prisms, m.p. 118—119°C.
Yield 32 g.

Elemental analysis, for $C_{14}H_{14}O_6 1NCl$

| | | | |
|---|---|---|---|
| Calcd.: | C, 51.31; | H, 4.31; | N, 4.27 |
| Found: | C, 51.24; | H, 4.26; | N, 4.16 |

Reference Example 14

63 g of Methyl 4-acetylamino-2-hydroxybenzoate were reacted in the same manner as in Reference Example 13. The product was subjected to column chromatography on silica gel and separated into two fractions. The crystals obtained from the first fraction were recrystallized from methanol to give 6-acetylamino-4′,5′-dihydrospiro[benzo[b]-furan]I-2′,3-dione as colourless plates, m.p. 220—234°C.
Yield 1.4 g.

Elemental analysis, for $C_{13}H_{11}O_5N$

| | | | |
|---|---|---|---|
| Calcd.: | C, 59.77; | H, 4.24; | N, 5.36 |
| Found: | C, 59.71; | H, 4.21; | N, 5.28 |

From the second fraction there was obtained $\alpha$-[(5-acetylamino-2-methoxycarbonylphenyl)oxy]-$\gamma$-butyrolactone as a pale yellow oil. Yield: 35 g. This only product can be subjected to the subsequent reaction step without further purification.

NMR($CDCl_3$) $\delta$: 2,10 (3H, s, $NCOCH_3$), 2.65 (2H, m, $CH_2$), 3.83 (3H, s, $COOCH_3$), 4.45 (2H, m, $OCH_2$), 4.98 (1H, t, OCHCO), 7.09 (1H, d, aromatic ring H), 7.66 (1H, s, aromatic ring H), 7.73 (1H, d, aromatic ring H).

Reference Example 15

24.4 g of $\alpha$-[(2-Carboxy-6-methylphenyl)oxy]-$\gamma$-butyrolactone were added to 120 ml of fuming nitric acid at a temperature not higher than —40°C. The reaction solution was poured into ice water, and the precipitating crystals were collected by filtration, washed with water and dried. The crystals were recrystallized from methanol. By the above procedure there was obtained $\alpha$-[(2-carboxy-6-methyl-4-nitrophenyl]-$\gamma$-butyrolactone as pale yellow prisms, m.p. 210°C (decomp.) Yield: 20.3 g.

Elemental analysis, for $C_{12}H_{11}O_7N$

| | | | |
|---|---|---|---|
| Calcd.: | C, 51.25; | H, 3.94; | N, 4.98 |
| Found: | C, 51.16; | H, 3.93; | N, 4.82 |

Reference Example 16

3.04 g of Methyl salicylate were reacted with $\alpha$-bromo-$\gamma$-butyrolactone in the same manner as in the corresponding step of Reference Example 13. The product was recrystallized from methanol to afford 3.3 g of $\alpha$-[(2-methoxycarbonylphenyl)oxy]-$\gamma$-butyrolactone as colourless needles melting at 62—87°C.

Elemental analysis, for $C_{12}H_{12}O_5$

| | | |
|---|---|---|
| Calcd.: | C, 61.01; | H, 5.12 |
| Found: | C, 60.98; | H, 4.99 |

Reference Example 17

A mixture of 1.3 g. of $\alpha$-[(2-carboxyphenyl)oxy]l-$\gamma$-butyrolactone, 15 ml. of acetic anhydride and 3 ml of triethylamine was stirred in nitrogen gas streams at 140°C for 3.5 hours, at the end of which time the solvents were distilled off under reduced pressure. Column chromatography was carried out on the residue using 32.5 g. of silica gel and carbon tetrachloride-acetone (10:1). The fraction corresponding to the contemplated compound was taken, concentrated under reduced pressure and recrystallized from *n*-hexane-ethylacetate (3:1). By the above procedure there were obtained 633 mg. of 4′,5′-dihydrospiro[benzo[b]-furan-2(3H), 3′(2′H)-furan]-2′,3-dione as colourless needles melting at 111—111.5°C.

Elemental analysis, for $C_{11}H_8O_4$
Calcd.: C, 64.70; H, 3.95
Found: C, 64.74; H, 3.70

Reference Examples 18—29

The following compounds were produced by a procedure similar to that described in Reference Example 17.

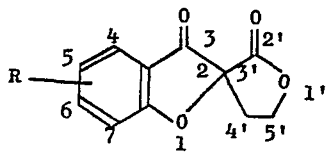

| Reference Example No. | Compound R | m.p. (°C) | Elemental analysis: Molecular formula | (Upper rank: Calcd.) Lower rank: Found) C | H | N |
|---|---|---|---|---|---|---|
| 18 | 5-Cl | 132.5—133 | $C_{11}H_7ClO_4$ | 55.36<br>55.49 | 2.96<br>2.79 | |
| 19 | 7-$CH_3$ | 103—104 | $C_{12}H_{10}O_4$ | 66.05<br>66.31 | 4.62<br>4.63 | |
| 20 | 5-Cl, 7-Cl | 157—159 | $C_{11}H_6Cl_2O_4$ | 48.38<br>48.47 | 2.21<br>2.14 | |
| 21 | 5-$OCH_2Ph$ | 138—139 | $C_{18}H_{14}O_5$ | 69.67<br>69.67 | 4.55<br>4.39 | |
| 22 | 6-$OCH_3$ | 106—108 | $C_{12}H_{10}O_5$ | 61.54<br>61.62 | 4.30<br>4.22 | |
| 23 | 5-$OCH_3$ | 120—122 | $C_{12}H_{10}O_5$ | 61.54<br>61.31 | 4.30<br>4.24 | |
| 24 | 5-$COCH_3$ | 132—134 | $C_{13}H_{10}O_5$ | 63.41<br>63.57 | 4.09<br>4.02 | |
| 25 | 4-$OCOCH_3$ | 135—137 | $C_{13}H_{10}O_6$ | 59.54<br>59.55 | 3.84<br>3.68 | |
| 26 | 5,6- 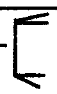 | 168—170 | $C_{15}H_{10}O_4$ | 70.86<br>70.83 | 3.96<br>3.63 | |
| 27 | 5-$NO_2$, 7-$CH_3$ | 127—130 | $C_{12}H_9O_6N$ | 54.76<br>55.00 | 3.45<br>3.24 | 5.32<br>5.36 |
| 28 | 5-$C_6H_{13}$ | oil | $C_{17}H_{20}O_4$ | 70.81<br>71.14 | 6.99<br>6.99 | |
| 29 | 5-$CH(CH_3)_2$ | 71 | $C_{14}H_{14}O_4$ | 68.28<br>68.39 | 5.73<br>5.67 | |

(Ph represents phenyl).

Reference Example 30

A mixture of 23 g. of $\alpha$-[(5-acetylamino-4-chloro-2-methoxycarbonylphenyl)oxy]-$\gamma$-butyrolactone, 46 ml. of triethylamine and 230 ml. of acetic anhydride was heated at 120°C for 5 hours. The solvents were evaporated off under reduced pressure, and the residue was poured into ice-water. The precipitating crystals were collected by filtration, washed with water and dried, followed by recrystallization from ethyl acetate to give 6-diacetylamino-5-chloro-4′,5′-dihydrospiro[benzo[b]furan-2(3H), 3′(2′H)-furan]2′,3-dione melting at 181—185°C. Yield: 6.8 g.

Elemental analysis, for $C_{15}H_{12}O_6NCl$

| | | | |
|---|---|---|---|
| Calcd.: | C, 53.34; | H, 3.58; | N, 4.15 |
| Found: | C, 53.08; | H, 3.49; | N, 4.12 |

Reference Example 31

39 g. of α-[(5-Acetylamino-2-methoxycarbonylphenyl)oxy]-γ-butyrolactone were reacted in the same manner as in Reference Example 30, whereby 1.8 g of 6-acetylamino-4',5'-dihydrospiro[benzo[b]furan-2(3H), 3'(2'H)-furan]-2',3-dione melting at 220—234°C. and 2.7 g. of 6-diacetylamino-4',5'-dihydrospiro[benzo[b]furan-2(3H), 3'(2'H)-furan]-2',3-dione melting at 178°C. were obtained.

Elemental analysis, for $C_{15}H_{13}O_6N$

| | | | |
|---|---|---|---|
| Calcd.: | C, 59.40; | H, 4.32; | N, 4.62 |
| Found: | C, 59.49; | H, 4.21; | N, 4.34 |

Reference Example 32

1.1 g. of α-[(2-Methoxycarbonylphenyl)oxy]-γ-butyrolactone were treated as in Reference Example 17 and the product was recrystallized from ethyl acetate-*n*-hexane. By the above procedure there was obtained 4',5'-dihydrospiro[benzo[b]furan-2(3H),3'(2'H)-furan]-2',3-dione as colourless needles, m.p. 111—111.5°C. Yield: 330 mg.

Reference Example 33

To a solution of 0.408 g. of 4',5'-dihydrospiro[benzo[b]furan-2(3H), 2'(2'H)-furan]-2',3-dione in 3 ml. of concentrated sulpuric acid was added a mixture of 0.35 ml. of nitric acid (d = 1.42) and 0.36 ml. of concentrated sulphuric acid, dropwise under ice-cooling, and the resulting mixture was stirred for 2 hours. The reaction mixture was poured into ice-water and the precipitated crystals were collected by filtration, washed with water, dried and recrystallized from ethyl acetate. By the above procedure there were obtained colourless needles of 4',5'-dihydro-5-nitrospiro[benzo[b]furan-2(3H),3'(2'H)-furan]-2',3-dione. m.p. 199—220°C.

Elemental analysis, for $C_{11}H_7NO_6$

| | | | |
|---|---|---|---|
| Calcd.: | C, 53.02; | H, 2.83; | N, 5.62 |
| Found: | C, 52.89; | H, 2.65; | N, 5.55 |

Reference Example 34

A mixture of 4',5'-dihydrospiro[benzo[b]furan-2(3H), 3'(2'H)-furan]-2',3-dione (3 g.) and chlorosulphonic acid was stirred at room temperature and, then, at 40°C for 1.5 hours. The reaction mixture was poured into ice-water, whereupon white crystals were separated. The crystals were dissolved in tetrahydrofuran, aqueous ammonia (2.2 ml.) was added and the mixture was stirred under ice-cooling for 5 minutes. The powdery precipitates were filtered off, the filtrate was concentrated under reduced pressure and the residue was recrystallized from ethanol-water. By the above procedure, there was obtained 5-sulphamoyl-4,',5'-dihydrospiro[benzo[b]furan-2(3H),3'(2'H)-furan]-2',3-dione as colourless needles, m.p. 202—215°C. Yield: 2.8 g.

Elemental analysis, for $C_{11}H_9O_6NS$

| | | | |
|---|---|---|---|
| Calcd.: | C, 46.64; | H, 3.20; | N, 4.95 |
| Found: | C, 46.39; | H, 3.14; | N, 4.87 |

Example 1

A mixture of 1.75 g. of 4',5'-dihydrospiro[benzo[b]furan-2(3H),3'(2'H)-furan]-2',3-dione, 552 mg. of sodium chloride and 9 ml. of dimethylsulphoxide was stirred in nitrogen gas streams at 155°C for 2 hours. The reaction mixture was poured into ice-water (ca 150 ml.) and the precipitate was recovered by filtration, washed with water and recrystallized from ethanol-water (3:2). By the above procedure there were obtained 1.21 g. of spiro[benzo[b]-furank-2(3H),1'-cyclopropane]-3-one as colourless needles melting at 89—90.5C.

Elemental analysis, for $C_{10}H_8O_2$

| | | |
|---|---|---|
| Calcd.: | c, 74.99; | H, 5.03 |
| Found: | C, 74.71; | H, 4.96 |

Examples 2—15

The following compounds were produced by a procedure similar to that described in Example 1.

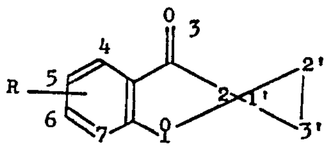

| Example No. | Compound R | Melting Point (°C) | Elemental Analysis | | | |
|---|---|---|---|---|---|---|
| | | | Molecular formula | Upper rank: Calcd. Lower rank: Found C | H | N |
| 2 | 5-Cl | 120–121 | $C_{10}H_7ClO_2$ | 61.71<br>61.68 | 3.63<br>3.50 | |
| 3 | 7-$CH_3$ | 126–129 | $C_{11}H_{10}O_2$ | 75.84<br>75.76 | 5.79<br>5.80 | |
| 4 | 5-Cl<br>7-Cl | 116–118 | $C_{10}H_6Cl_2O_2$ | 52.43<br>52.65 | 2.64<br>2.61 | |
| 5 | 5-$NO_2$ | 107–110 | $C_{10}H_7NO_4$ | 58.54<br>58.85 | 3.44<br>3.50 | 6.83<br>6.68 |
| 6 | 5-$OCH_2Ph$ | 114–116 | $C_{17}H_{14}O_3$ | 76.67<br>76.53 | 5.30<br>5.18 | |
| 7 | 6-$OCH_3$ | 95–97 | $C_{11}H_{10}O_3$ | 69.46<br>69.35 | 5.30<br>5.30 | |
| 8 | 5-$OCH_3$ | 86–88 | $C_{11}H_{10}O_3$ | 69.46<br>69.31 | 5.30<br>5.13 | |
| 9 | 5-$COCH_3$ | 100–103 | $C_{12}H_{10}O_3$ | 71.28<br>71.07 | 4.99<br>4.82 | |
| 10 | 4-$OCOCH_3$ | 68–71 | $C_{12}H_{10}O_4$ | 66.05<br>65.89 | 4.62<br>4.52 | |
| 11 | 5-$SO_2NH_2$. | 228–239<br>(sublimation) | $C_{10}H_9O_4NS$ | 50.20<br>50.19 | 3.79<br>3.71 | 5.86<br>5.79 |
| 12 | 5-$NO_2$<br>7-$CH_3$ | 160–162 | $C_{11}H_9O_4N$ | 60.27<br>60.17 | 4.14<br>4.14 | 6.39<br>6.48 |
| 13 | 5-$CH(CH_3)_2$ | b.p. 113<br>(0.4mmHg) | $C_{13}H_{14}O_2$ | 77.20<br>77.43 | 6.98<br>7.11 | |
| 14 | 6-NHAc | 171–178 | $C_{12}H_{11}O_3N$ | 66.35<br>66.30 | 5.10<br>5.00 | 6.45<br>6.20 |
| 15 | 5-Cl, | 185–188 | $C_{12}H_{10}O_3NCl$ | 57.27<br>57.03 | 4.01<br>3.86 | 5.57<br>5.46 |

(Ph represents phenyl and Ac represents acetyl)

Example 16

4′,5′-Dihydrospiro[naphtho[2,3-b]furan-2(3H),3′(2′H)-furan]-2′,3-dione (1.2 g.) was reacted in the same manner as in Example 1 and the reaction product was recrystallized from methanol. By the above procedure there was obtained spiro[naphtho[2,3-b]furan-2(3H),1′-cyclopropane]-3-one as colourless needles, m.p. 127—129°C. Yield: 0.75 g.

Elemental analysis, for $C_{14}H_{10}O_2$

Calcd.: C, 79.98; H, 4.79

Found: C, 79.89; H, 4.65

Example 17

5-Hexyl-4',5'-dihydrospiro[benzo[b]furan-2(3H),3'(2'H)-furan]-2',3-dione was decarboxylated in the same manner as in Example 1 to yield 5-hexylspiro[benzo[b]furan-2(3H),1'-cyclopropane]-3-one as a pale yellowish oil.

$IR_{max}^{film}$ $cm^{-1}$: 1700(CO).

NMR ($CDCl_3$) $\delta$: 0.87 (3H, t, $CH_3$), 1.36 (8H, b, $CH_2$), 1.59 (4H, m, cyclopropane), 2.63 (2H, t, nuclear $CH_2$), 7.02 (1H, d, nuclear H), 7.40 (1H, d, nuclear H), 7.48 (1H, s, nuclear H).

Elemental analysis, for $C_{16}H_{20}O_2$

| | | |
|---|---|---|
| Calcd.: | C, 78.65; | H, 8.25 |
| Found: | C, 78.37; | H, 8.36 |

Example 18

0.94 g. of spiro[benzo[b]furan-2(3H),1'-cyclopropanel]-3-one was dissolved, in 30 ml. of acetic anhydride, and, at 60—70°C, 5.6 g. of copper nitrate were added. The resulting solution was stirred overnight. The reaction mixture was poured into ice-water and extracted with ethyl acetate. The extract was washed with water, dried and distilled to remove the solvent. The residue was fractionated by column chromatography on silica gel into two fractions:

(1) The first fraction was recrystallized from ethyl acetate-*n*-hexane to yield 5-nitrospiro[benzo[b]furan-2(3H),1'-cyclopropane]-3-one as colourless prisms melting at 107—110°C.

Elemental analysis, for $C_{10}H_7NO_4$

| | | | |
|---|---|---|---|
| Calcd.: | C, 58.54; | H, 3.44; | N, 6.83 |
| Found: | C, 58.85; | H, 3.50; | N, 6.68 |

(2) The second fraction was recrystallized from ethyl acetate-hexane to yield 7-nitrospiro[benzo[b]furan-2(3H),1'-cyclopropane]-3-one as colourless needles melting at 131—134°C.

Elemental analysis, for $C_{10}N_7NO_4$

| | | | |
|---|---|---|---|
| Calcd.: | C, 58.54; | H, 3.44; | N, 6.83 |
| Found: | C, 58.42; | H, 3.37; | N, 6.65 |

Example 19

Spiro[benzo[b]furan-2(3H),1'-cyclopropane]-3-one (7.0 g.) was added in small portions to fuming nitric acid (70 ml.) previously cooled to −50°C to −60°C. After stirring for 20 minutes, the reaction mixture was poured into ice-water and the precipitated crystals were collected by filtration, washed with water and recrystallized from ethanol. By the above procedure there was obtained 5-nitrospiro[benzo[b]furan-2(3H),1'-cyclopropane]-3-one as colourless prisms, m.p. 107—110°C. Yield: 7.3 g. This product was in good agreement with the crystals obtained in Example 18.

The mother liquor resulting from the recrystallization was subjected to column chromatography on silica-gel for purification, and then recrystallized from methanol to afford 5,7-dinitrospiro[benzo[b]furan-2(3H),1'-cyclopropane]-3-one as pale yellow needles melting at 158—161°C.

Elemental analysis, for $C_{10}H_6O_6N_2$

| | | | |
|---|---|---|---|
| Calcd.: | C, 48.01; | H, 2.32; | N, 11.20 |
| Found: | C, 48.03; | H, 2.33; | N, 11.01 |

Example 20

5.4 g. of 6-Methoxyspiro[benzo[b]furan-2(3H),1'-cyclopropane]-3-one were dissolved in a mixture of 25 ml. acetic anhydride and 7 ml. of glacial acetic acid. While keeping the reaction temperature at 10—15°C, 3 ml. of fuming nitric acid (d = 1.52) were added dropwise to the mixture. After stirring for 30 minutes, the reaction mixture was poured into ice-water. The resulting precipitates were collected by filtration, washed with water and recrystallized from ethanol. By the above procedure, there was obtained 6-methoxy-5-nitrospiro[benzo[b]furan-2(3H),1'cyclopropane]-3-one as pale yellow prisms melting at 160—163°C. Yield: 4.5 g.

Elemental analysis, for $C_{11}H_9NO_5$

| | | | |
|---|---|---|---|
| Calcd.: | C, 56.17; | H, 3.86; | N, 5.96 |
| Found: | C, 56.44; | H, 3.76; | N, 5.80 |

Example 21

190 mg. of 6-Methoxyspiro[benzo[b]furan-2(3H),1′-cyclopropane]-3-one were added to 2 ml. of fuming nitric acid (d = 1.52) at —50°C while stirring. After 10 minutes, the reaction solution was poured into ice-water, and then extracted with ethyl acetate. The extract solution was washed with an aqueous solution of sodium bicarbonate, and then with a saturated saline solution, followed by drying over anhydrous sodium sulphate. Crystals obtained by evaporating the solvent were recrystallized from methanol. By the above procedure, there were obtained 20 mg. of 6-methoxy-5,7-dinitrospiro[benzo[b]furan-2(3H),1′-cyclopropane]-3-one as pale yellow plates melting at 121—124°C.

Elemental analysis, for $C_{11}H_8O_7N_2$

| | | | |
|---|---|---|---|
| Calcd.: | C, 47.15; | H, 2.88; | N, 10.00 |
| Found: | C, 46.86; | H, 2.79; | N, 9.83 |

Example 22

A solution of 5-nitrospiro[benzo[b]furan-2(3H),1′-cyclopropane]-3-one (7.2 g.) in ethanol was stirred in the presence of platinum dioxide and in hydrogen gas streams. After the hydrogen absorption had ceased, the catalyst was filtered off and a small amount of HCl-diethyl ether was added to the residue, followed by recrystallization from ethanol. By the above procedure there was obtained 5-aminospiro[benzo[b]furan-2(3H),1′-cyclopropane]-3-one hydrochloride as light-brown needles melting at 139—142°C.

Elemental analysis, for $C_{10}H_{19}O_2N{\cdot}HCl$

| | | | |
|---|---|---|---|
| Calcd.: | C, 56.75; | H, 4.76; | N, 6.62 |
| Found: | C, 56.67; | H, 4.83; | N, 6.67 |

Example 23

7-Nitrospiro[benzo[b]furan-2(3H),1′-cyclopropane]-3-one was reacted in the same manner as in Example 22 and the reaction product was recrystallized from ethanol. By the above procedure there was obtained 7-aminospiro[benzo[b]furan-2(3H),1′-cyclopropane]-3-one as pale brown crystals melting at 135.8°C.

Elemental analysis, for $C_{10}H_{19}O_2N$

| | | | |
|---|---|---|---|
| Calcd.: | C, 68.56; | H, 5.18; | N, 8.00 |
| Found: | C, 68.42; | H, 5.11; | N, 7.74 |

Example 24

1.0 g. of 6-Methoxy-5-nitrospiro[benzo[b]furan-2(3H),1′-cyclopropane]-3-one was reacted in the same manner as in Example 22, and the reaction product was recrystallized from ethanol. By the above procedure, there were obtained 415 mg. of 5-amino-6-methoxyspiro[benzo[b]furan-2(3H),1′-cyclopropane]-3-one as pale brown prisms melting at 175—177°C.

Elemental analysis, for $C_{11}H_{11}NO_3$

| | | | |
|---|---|---|---|
| Calcd.: | C, 64.38; | H, 5.40; | N, 6.83 |
| Found: | C, 64.39; | H, 5.49; | N, 6.71 |

Example 25

219 mg. of 7-Methyl-5-nitrospiro[benzo[b]furan-2(3H),1′-cyclopropane]-3-one were subjected to catalytic reduction as in Example 22, and the reaction product was recrystallized from ethanol-water. By the above procedure there were obtained 74 mg. of 5-amino-7-methylspiro[benzo[b]furan-2(3H),1′-cyclopropane]-3-one as yellow needles melting at 138—141°C.

Elemental analysis, for $C_{11}H_{11}O_2N$

| | | | |
|---|---|---|---|
| Calcd.: | C, 69.82; | H, 5.86; | N, 7.40 |
| Found: | C, 69.66; | H, 5.71; | N, 7.43 |

Example 26

250 mg. of 5,7-Dinitrospiro[benzo[b]furan-2(3H),1′-cyclopropane]-3-one, 50 mg. of platinum dioxide and 20 ml. of ethanol were stirred in a stream of hydrogen for 1.25 hour under atmospheric pressure. Oxalic acid was added to the reaction mixture, and the catalyst was removed by filtration. The

filtrate was concentrated under reduced pressure until its volume became about 3 ml. Ether was added to the concentrate, and the resulting powder was collected by filtration. The powder was dissolved in ethanol. To the ethanolic solution was added activated charcoal for decolouration, followed by the addition of ether. The precipitating powder was collected by filtration to obtain 5,7-diaminospiro[benzo[b]furan-2(3H),1'-cyclopropane]-3-one.1/2 oxalate.monohydrate as a yellish brown powder.

Elemental analysis, for $C_{10}H_{10}O_2N_2 \cdot \frac{1}{2}(COOH)_2 \cdot H_2O$

| | | | |
|---|---|---|---|
| Calcd.: | C, 52.17; | H, 5.17; | N, 11.06 |
| Found: | C, 52.12; | H, 4.69; | N, 10.87 |

The use of hydrochloric acid instead of oxalic acid in the above procedure gives 5,7-diaminospiro[benzo[b]furan-2(3H),1'cyclopropane]-3-one·hydrochloride·monohydrate melting at a temperature not lower than 300°C.

Elemental analysis, for $C_{10}H_{10}O_2N_2 \cdot HCl \cdot H_2O$

| | | | |
|---|---|---|---|
| Cacld.: | C, 49.08; | H, 5.35; | N, 11.45 |
| Found: | C, 48.80; | H, 5.13; | N, 11.64 |

## Example 27

Carbobenzyloxy chloride (30% toluene solution, 7 g.) was added under ice-cooling to a solution of 5-aminospiro[benzo[b]furan-2(3H),1'-cyclopropane]-3-one (1.35 g.) in pyridine (13.5 ml.) and the resulting mixture was stirred for one hour. The reaction mixture was poured into ice-hydrochloric acid (14 ml.) and extracted with ethyl acetate. The extract was washed with water, dried and concentrated to remove the solvent. The residue was recrystallized from ethanol. By the above procedure there was obtained 5-benzyloxycarbonylaminospiro[benzo[b]furan-2(3H),1'-cyclopropane]-3-one as pale yellow needles, m.p. 118—119°C. Yield: 1.57 g.

Potassium hydroxide powder (0.57 g.) and methyl iodide (1 ml.) were added to a solution of this product in acetone (30 ml.) and the resulting mixture was stirred under ice-cooling for 30 minutes and, then, at room temperature for 4 hours. Dilute hydrochloric acid was added to this reaction mixture, followed by, extraction with ethyl acetate. The extract was washed with water, dried and distilled under reduced pressure to remove the solvent. The residue was chromatographed on a column of silica gel and the fraction eluted with chloroform was recrystallized from ethanol. By the above procedure there was obtained 5-(N-benzyloxycarbonyl-N-methylamino)spiro[benzo[b]furan-2(3H),1'-cyclopropane]-3-one as colourless needles melting at 79—81°C. Yield: 1.44 g.

This product was dissolved in methanol, (129 ml.), and, in the presence of 5% palladium-on-carbon, the solution was stirred in hydrogen gas streams for 30 minutes. The catalyst was filtered off, the filtrate was concentrated under reduced pressure and the residue was dissolved in ethanol, followed by the addition of HCl-diethyl ether. By the above procedure there was obtained 5-methylaminospiro[benzo[b]furan-2(3H),1'-cyclopropane]-3-one hydrochloride as yellow needles melting at 141—144°C.

Elemental analysis, for $C_{11}H_{11}O_2N \cdot HCl \cdot \frac{1}{2}H_2O$

| | | | |
|---|---|---|---|
| Calcd.: | C, 56.29; | H, 5.58; | N, 5.97 |
| Found: | C, 56.38; | H, 5.15; | N, 6.07 |

## Example 28

5-Aminospiro[benzo[b]furan-2(3H),1'-cyclopropane]-3-one (1.75 g.) and 37% formalin (14 ml.) were dissolved in acetonitrile, and, under ice-cooling, lithium cyanborohydride (1.52 g.) was added to the solution portionwise. The mixture was stirred at room temperature for 40 minutes, after which it was neutralized with acetic acid and then stirred for 2.5 hours. The solvent was distilled off under reduced pressure, an aqueous solution of sodium hydroxide was added to the residue and the mixture was extracted with chloroform. The extract was washed with water, dried and concentrated to remove the solvent. The residue was chromatographed on silica gel, elution being carried out with chloroform. To the eluate was added HCl-diethyl ether, followed by recrystallization from ethanol. By the above procedure, there was obtained 5-dimethylaminospiro[benzo[b]furan-2(3H),1'-cyclopropane]-3-one hydrochloride as light-brown needles melting at 136—140°C. Yield: 0.546 g.

NMR ($D_2O$) $\delta$: 1.67 (2H, m, $CH_2$), 1.93 (2H, m, $CH_2$), 3.37 (6H, s, $CH_3$), 7.43 (1H, d, aromatic ring H), 7.93 (2H, m, aromatic ring H).

Elemental analysis, for $C_{12}H_{13}O_2H \cdot NCl$

| | | | |
|---|---|---|---|
| Calcd.: | C, 60,13; | H, 5.89; | N, 5.85 |
| Found: | C, 60.19; | H, 5.72; | N, 6.00 |

Example 29

A mixture of 35 g. of 5-nitrospiro[benzo[b]furan-2(3H),1'cyclopropane]-3-one, 60 ml. of 37% formalin, 30 ml. of acetic acid, 3 g. of platinum dioxide and 500 ml. of ethanol was subjected to reduction at room temperature under a hydrogen pressure of 20 kg./cm$^2$. After stopping the hydrogen absorption, the catalyst was removed by filtration, and the filtrate was concentrated under reduced pressure. The concentrate was dissolved in chloroform, and washed with 2N NaOH and then with water, followed by drying. Chloroform was removed by evaporation under reduced pressure, and the resulting oily substance was crystallized from ethanol to obtain 26 g. of 5-dimethylamino[b]spiro[benzo[b]furan-2(3H),1'cyclopropane]-3-one as yellow cubic crystals melting at 96.5—97.5°C.

Elemental analysis, for $C_{12}H_{13}O_2N$

| | | | |
|---|---|---|---|
| Cacld.: | C, 70.91; | H, 6.45; | N, 6.89 |
| Found: | C, 71.06; | H, 6.39; | N, 6.71 |

Example 30

5-Aminospiro[benzo[b]furan-2(3H),1'-cyclopropane]-3-one (1.75 g.) and acetaldehyde (3 ml.) were dissolved in methanol (105 ml.). The methanolic solution was stirred for 22 hours in a hydrogen stream in the presence of platinum dioxide. After removal of the catalyst by filtration, the solvent was evaporated off, and the residue was subjected to column-chromatography on silica gel, using carbon tetrachloride-ethyl acetate (10:1) as the eluent. The first fraction was converted to the hydrochloride with ether saturated with hydrogen chloride, the product being recrystallized from ethanol-ether. By the above procedure, there was obtained 5-diethylaminospiro[benzo[b]furan-2(3H),1'-cyclopropane]-3-one hydrochloride as pale yellow needles melting at 172—176°C. Yield: 0.86 g.

Elemental analysis, for $C_{14}H_{17}O_2H{\cdot}HCl$

| | | | |
|---|---|---|---|
| Calcd.: | C, 62.80; | H, 6.78; | N, 5.23 |
| Found: | C, 62.79; | H, 6.85; | N, 5.10 |

The second fraction was converted to the hydrochloride with ether saturated with hydrogen chloride, the product being recrystallized from ethanol-ether to yield 5-ethylaminospiro[benzo[b]furan-2(3H),1'-cyclopropane]-3-one hydrochloride 1/4 hydrate as pale yellow needles melting at 155—160°C. Yield: 0.129 g.

Elemental analysis, for $C_{12}H_{13}O_2N{\cdot}HCl{\cdot}1/4H_2O$

| | | | |
|---|---|---|---|
| Cacld.: | C, 59.02; | H, 5.98; | N, 5.73 |
| Found: | C, 58.94; | H, 5.86; | N, 5.73 |

Example 31

A mixture of 5-aminospiro[benzo[b]furan-2(3H),1'-cyclopropane]-3-one (3.0 g.), 1,4-dibromobutane (3.7 g.), sodium bicarbonate (2.89 g.) and N,N-dimethylformamide (150 ml.) was heated under reflux for one hour. The reaction mixture was diluted with water and extracted with ethyl acetate. The extract was washed with water, dried and concentrated to remove the solvent. The residue was chromatographed on silica gel, elution being carried out with chloroform. The first fraction thus obtained was distilled under reduced pressure to recover yellow crystals (1.74 g.). Following the addition of HCl-diethyl ether, the product was recrystallized from ethanol. By the above procedure there were obtained yellow needles of 5-(1-pyrrolidinyl)spiro[benzo[b]furan-2(3H),1'-cyclopropane]-3-one hydrochloride. m.p. 136°C.

Elemental analysis, for $C_{14}H_{15}O_2N.HCl$

| | | | |
|---|---|---|---|
| Calcd.: | C, 63.27; | H, 6.07; | N, 5.27 |
| Found: | C, 63.26; | H, 6.10; | N 5.26 |

Example 32

A suspension of 5-aminospiro[benzo[b]furan-2(3H),1'-cyclopropane]-3-one (2.62 g.), bis(2-iodoethyl) ether (5.4 g.) and sodium bicarbonate (3.75 g.) in N,N-dimethylformamide (150 ml.) was stirred at 120—140°C for 2.5 hours. The reaction solution was poured into water and extracted with ethyl acetate. The extract was washed with water and dried and the solvent was removed by

evaporation. The residue was subjected to column-chromatography on silica-gel using chloroform-ethanol (99:1) as the eluent. The eluate was concentrated by evaporation of the solvent under reduced pressure to give yellow crystals (1.12 g.), to which HCl-ether was added, and was then recrystallized from ethanol-ether to obtain 5-morpholinospiro[benzo[b]furan-2(3H),1′-cyclopropane]-3-one hydrochloride as pale brown needles, melting at 128—131°C. Yield: 0.927 g.

Elemental analysis, for $C_{14}H_{15}O_3N{\cdot}HCl$

| | | | |
|---|---|---|---|
| Calcd.: | C, 59.68; | H, 5.73; | N, 4.97 |
| Found: | C, 59.59; | H, 5.60; | N, 4.95 |

Example 33

5-Aminospiro[benzo[b]furan-2(3H),1′-cyclopropane]-3-one (1.75 g.) was allowed to react with N-benzyl-β,β′-diiododiethylamine (6.8 g.) and sodium bicarbonate (4 g.) in the same manner as in Example 32 to obtain 5-(4-benzyl-1-piperazinyl)spiro[benzo[b]furan-2(3H),1′-cyclopropane]-3-one as yellow needles melting at 125—125.5°C. Yield: 0.831 g.

Elemental analysis, for $C_{21}H_{22}O_2N_2$

| | | | |
|---|---|---|---|
| Calcd.: | C, 72.42; | H, 6.63; | N, 8.38 |
| Found: | C, 75.26; | H, 6.78; | N, 8.41 |

Example 34

5-Aminospiro[benzo[b]furan-2(3H),1′cyclopropane]-3-one (1.75 g.) was allowed to react with N-ethyl-β,β′-diiododiethylamine (5.84 g.) and sodium bicarbonate (4 g.) in the same manner as in Example 32 to obtain 5-(4-ethyl-1-piperazinyl)spiro[benzo[b]furan-2(3H),1′-cyclopropane]-3-one oxalate 1/2 hydrate as yellow needles melting at 175—179°C.

Elemental analysis, for $C_{16}H_{20}O_2N_2{\cdot}C_2H_2O_4{\cdot}\frac{1}{2}H_2O$

| | | | |
|---|---|---|---|
| Calcd.: | C, 58.20; | H, 6.24; | N, 7.54 |
| Found: | C, 58.00; | H, 6.56; | N, 7.24 |

Example 35

5-Aminospiro[benzo[b]furan-2(3H),1′-cyclopropane]-3-one (0.875 g.) was acetylated with acetic anhydride (7 ml.) and acetic acid (7 ml.) and the acylation product was recrystallized from ethanol. By the above procedure there was obtained 5-acetylaminospiro[benzo[b]furan-2(3H),1′-cyclopropane]-3-one as yellow prisms melting at 211—212°C. Yield: 0.426 g.

Elemental analysis, for $C_{12}H_{11}O_3N$

| | | | |
|---|---|---|---|
| Calcd.: | C, 66.35; | H, 5.10; | N, 6.45 |
| Found: | C, 66.37; | H, 5.12; | N, 6.38 |

Example 36

To a solution of 5-aminospiro[benzo[b]furan-2(3H),1′-cyclopropane]-3-one (0.519 g.) in pyridine (5 ml.) was added methanesulphonyl chloride (0.28 ml.) under ice-cooling, followed by stirring. The reaction mixture was poured into cooled dilute hydrochloric acid and extracted with ethyl acetate. The extract was washed with water, dried and concentrated to remove the solvent. The residue was recrystallized from ethanol. By the above procedure there was obtained 5-methylsulphonylaminospiro[benzo[b]furan-2(3H),1′-cyclopropane]-3-one as colourless needles melting at 152—154°C. Yield 0.38 g.

Elemental analysis, for $C_{11}H_{11}O_4NS$

| | | | | |
|---|---|---|---|---|
| Calcd.: | C, 52.16; | H, 4.38; | N, 5.53; | S, 12.66 |
| Found: | C, 52.20; | H, 4.37; | N, 5.32; | S, 12.56 |

Example 37

A 10% aqueous solution of sodium hydroxide was added to 4-acetoxyspiro[benzo[b]furan-2(3H),1′-cyclopropane]-3-one, and the resulting mixture was stirred at room temperature. The reaction mixture was made acidic with hydrochloric acid and extracted with ethyl acetate. The extract was washed with water, dried and distilled to remove the solvent. The residue was recrystallized from petroleum ether. By the above procedure there was obtained 4-hydroxyspiro[benzo[b]furan-2(3H),1′-cyclopropane]-3-one as yellow needles, m.p. 100—109°C.

Elemental analysis, for $C_{10}H_8O_3$

| | | |
|---|---|---|
| Calcd.: | C, 68.18; | H, 4.58 |
| Found: | C, 68.38; | H, 4.42 |

Example 38

To a solution of 1.09 g. of 6-acetylaminospiro[benzo[b]furan-2(3H),1'-cyclopropane]-3-one in 50 ml. of methanol was added 0.8 g. of potassium hydroxide, and the resulting mixture was refluxed for 0.5 hour. The solvent was evaporated off under reduced pressure. Water was added to the residue, and the precipitating crystals were collected by filtration, washed with water and dried. The crystals were recrystallized from methanol to obtain 6-aminospiro[benzo[b]furan-2(3H),1'-cyclopropane]-3-one as colourless prisms melting at 188—189°C.

Elemental analysis, for $C_{10}H_9O_2N$

| | | | |
|---|---|---|---|
| Calcd.: | C, 68.56; | H, 5.18; | N, 8.00 |
| Found: | C, 68.34; | H, 5.05; | N, 7.88 |

Example 39

6-Acetylamino-5-chlorospiro[benzo[b]furan-2(3H),1'-cyclopropane]-3-one (1.8 g.) was reacted in the same manner as in Example 38 and the reaction product was recrystallized from methanol. By the above procedure, there was obtained 6-amino-5-chlorospiro[benzo[b]furan-2(3H),1'-cyclopropane]-3-one as yellow plates, m.p. 201°C. Yield: 1.5 g.

Elemental analysis, for $C_{10}H_8O_2NCl$

| | | | |
|---|---|---|---|
| Calcd.: | C, 57.29; | H, 3.85; | N, 6.68 |
| Found: | C, 57.24; | H, 3.74; | N, 6.67 |

Example 40

5-Benzyloxyspiro[benzo[b]furan-2(3H),1'-cyclopropane]-3-one (3.3 g.) was debenzylated by catalytic reduction in methanol. By this procedure, there was obtained 5-hydroxyspiro[benzo[b]furan-2(3H),1'-cyclopropane]-3-one as pale yellow needles, m.p. 180—185°C. Yield: 1.8 g.

Elemental analysis, for $C_{10}H_8O_3$

| | | |
|---|---|---|
| Calcd.: | C, 68.18; | H, 4.58 |
| Found: | C, 68.12; | H, 4.44 |

Example 41

A mixture of 4-hydroxyspiro[benzo[b]furan-2(3H),1'-cyclopropane]-3-one (0.176 g.), potassium carbonate (0.276 g.), $\beta$-diethylaminoethyl chloride (0.215 g.) and N,N-dimethylformamide (5 ml.) was stirred at room temperature for 3 hours. The reaction mixture was diluted with water and extracted with ethyl acetate. The extract was washed with water, dried and distilled to remove the solvent. The residue was purified by column chromatography on silica gel, using chloroform as the eluent. The product was treated with HCl-saturated diethyl ether and the resulting hydrochloride was recrystallized from ethanol-diethyl ether. By the above procedure there was obtained 4-(2-diethylaminoethyloxy)spiro[benzo[b]furan-2(3H),1'-cyclopropane]-3-one hydrochloride as colourless needles, m.p. 160—168°C. Yield: 0.221 g.

Elemental analysis, for $C_{16}H_{21}O_3N \cdot HCl$

| | | | |
|---|---|---|---|
| Calcd.: | C, 61.63; | H, 7.11; | N, 4.49 |
| Found: | C, 61.38; | H, 7.23; | N, 4.38 |

Example 42

5-Hydroxyspiro[benzo[b]furan-2(3H),1'-cyclopropane]-3-one (1.06 g.) was reacted in the same manner as in Example 41 to obtain 5-(2-diethylaminoethyloxy)spiro[benzo[b]furan-2(3H),1'-cyclopropane]-3-one as a colourless oil. Nuclear magnetic resonance spectrum ($\delta$, in deuteriochloroform): 1.07 (6H, t, $CH_3$), 1.63 (4H, m, 2',3'-$CH_2$), 2.64 (4H, q, $NCH_2CH_3$), 2.88 (2H, t, $NCH_2CH_2O$), 4.04 (2H, t, $CH_2O$), 6.95—7.40 (3H, m, aromatic ring H).

Example 43

5-Aminospiro[benzo[b]furan-2(3H),1'-cyclopropane]-3-one (0.747 g.) and calcium carbonate (0.47 g.) were suspended in a mixture of carbon tetrachloride (20 ml.) and methylene chloride (5 ml.).

The suspension was cooled to −17°C, and then bromine (0.22 ml.) was added dropwise thereto, followed by stirring for 45 minutes. The reaction mixture was poured into ice-water, and then extracted with ethyl acetate. The extract was washed with water and dried. The solvent was evaporated off, and the residue was recrystallized from ethanol-water. By the above procedure there was obtained 5-amino-4-bromospiro[benzo[b]furan-2(3H),1′-cyclopropane]-3-one as yellow needles melting at 167—170°C. Yield: 0.6 g.

Elemental analysis, for $C_{10}H_8O_2NBr$

| | | | |
|---|---|---|---|
| Calcd.: | C, 47.27; | H, 3.19; | N, 5.51 |
| Found: | C, 47.58; | H, 3.12; | N, 5.64 |

Example 44

A suspension of 5-dimethylaminospiro[benzo[b]furan-2(3H),1′-cyclopropane]-3-one (0.455 g.) and calcium carbonate (0.246 g.) in carbon tetrachloride (10 ml.) was reacted in the same manner as in Example 43 to obtain 4-bromo-5-dimethylaminospiro[benzo[b]furan-2(3H),1′-cyclopropane]-3-one as brown needles melting at 79—81°C. Yield: 0.213 g.

Elemental analysis, for $C_{12}H_{12}O_2NBr$

| | | | |
|---|---|---|---|
| Calcd.: | C, 51.08; | H, 4.29; | N, 4.97 |
| Found: | C, 50.87; | H, 4.13; | N, 5.03 |

Example 45

A solution of 5-aminospiro[benzo[b]furan-2(3H),1′-cyclopropane]-3-one (0.181 g.) and pyridine (0.083 mg.) in tetrahydrofuran (5 ml.) was cooled to −17°C. Iodobenzenedichloride (0.282 g.) which had been prepared by a conventional method and dissolved in tetrahydrofuran (1.5 ml.), was added dropwise to the solution over 50 minutes, followed by stirring for 1 hour. The reaction mixture was poured into ice-water and extracted with ethyl acetate. The extract was washed with water and dried, and the solvent was evaporated off. The residue was subjected to column-chromatography, using chloroform as the eluent. The first fraction was concentrated under reduced pressure to remove the solvent. By the above procedure, there was obtained 5-amino-4-chlorospiro[benzo[b]furan-2(3H),1′cyclopropane]-3-one as yellow crystals. Yield: 0.038 g.
Mass spectrum: $C_{12}H_{12}O_2NCl$, molecular ion peak (209)

Examples of preparations ready for administration

When the compound of this invention is intended for use as an anti-ulcer, types of suitable preparations can be exemplified as follows.

1. Tablet

| | | | |
|---|---|---|---|
| (1) | 5-Acetylspiro[benzo[b]furan-2 (3H),1′cyclopropane]-3-one | | 50 g. |
| (2) | Lactose | | 50 g. |
| (3) | Corn-starch | | 29 g. |
| (4) | Magnesium stearate | | 1 g. |
| | | 1000 tablets | 130 g. |

Components (1) and (2) and 17 g. of the corn-starch (3) were granulated together with a paste prepared from 7 g. of the corn-starch. To these granules were added the remaining 5 g. of the corn-starch and component (4). The mixture was then compressed by a tabletting machine to prepare 1000 tablets of 7 mm. diameter, each containing 50 mg. of (1).

2. Capsule

| | | | |
|---|---|---|---|
| (1) | 5-Dimethylaminospiro[benzo[b]furan-2(3H),1'-cyclopropane]-3-one | | 50 g. |
| (2) | Lactose | | 100 g. |
| (3) | Cellulose fine powder | | 45 g. |
| (4) | Magnesium stearate | | 5 g. |
| | | 1000 capsules | 200 g. |

All the materials were mixed and filled into 1000 capsules (gelatin capsule No. 3 defined in Japanese Pharmacopoeia, 8th edition) to prepare capsules each containing 50 mg. of (1).

**Claims**

1. A spiro compound of the formula:

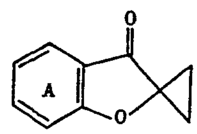

wherein Ring A represents a benzene ring or a naphthalene ring, the ring being unsubstituted or substituted by at least one of $C_{1-6}$ alkyl, nitro, halogen, amino, which may optionally itself be substituted, hydroxyl, which may optionally itself be substituted, acyl and sulfamoyl.

2. A compound according to claim 1, wherein Ring A is substituted by at least one of amino, mono- or dialkylamino and cycloamino.

3. A compound according to claim 2, which is in the form of a pharmaceutically acceptable acid addition salt.

4. A compound according to claim 1, wherein Ring A is benzene and the substituent of the benzene ring is di-$C_{1-4}$ alkylamino or $C_{2-6}$ alkanoyl.

5. A compound according to claim 4, wherein the substituted position is the 5-position of the benzene ring.

6. Spiro[benzo[b]furan-2(3H),1'-cyclopropane]-3-one.

7. 5-acetylspiro[benzo[b]furan-2(3H),1'-cyclopropane]-3-one.

8. Spiro-[naphtho[2,3-b]furan-2(3H),1'-cyclopropane]-3-one.

9. 5-Nitro-spiro[benzo[b]furan-2(3H),1'-cyclopropane]-3-one.

10. 5-Amino-spiro[benzo[b]furan-2(3H),1'-cyclopropane]-3-one.

11. 5-Methyl-aminospiro[benzo[b]furan-2(3H),1'-cyclopropane]-3-one.

12. 5-Dimethylaminospiro[benzo[b]furan-2(3H),1'-cyclopropane]-3-one.

13. A pharmaceutical composition which comprises (A), as an active ingredient, an effective amount of the spiro compound as defined in claim 1, and (B) a pharmaceutically acceptable carrier or diluent therefor.

14. A method of producing a spiro compound of the formula:

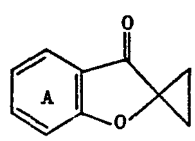

wherein Ring A represents a benzene ring or a naphthalene ring, the ring being unsubstituted or substituted by at least one of $C_{1-6}$ alkyl, nitro, halogen, amino, which may optionally itself be substituted, hydroxyl, which may optionally itself be substituted, acyl and sulphamoyl,

which method comprises decarboxylating a compound of the formula:

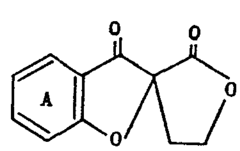

wherein Ring A is as defined above;

and optionally converting the compound (I) to an acid addition salt when at least one amino substituent is present on Ring A and to an alkali metal salt when at least one hydroxyl group is present on Ring A.

15. A method of producing a spiro compound of the formula:

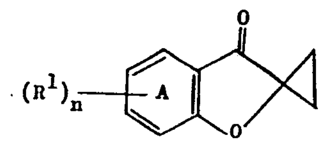

(Ic)

wherein Ring A is as defined above, $R^1$ is mono- or di-alkylamino and *n* is 1 or 2, which method comprises:
(1) subjecting a compound of the formula:

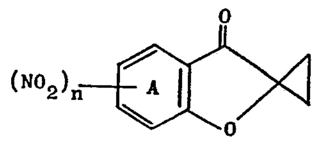

(Ia)

wherein *n* is as defined above,
to reduction, and then the resulting compound of the formula:

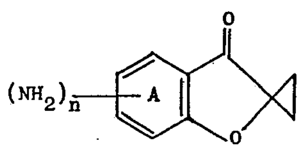

(Ib)

wherein *n* is as defined above,
to reductive alkylation or alkylation with an alkyl halide; or
(2) subjecting a compound of the formula (Ia) to reductive alkylation; and optionally converting the compound (Ic) to an acid addition salt thereof.

16. The compound (I) or acid addition salts thereof as claimed in claims 1—12, or a pharmaceutical composition as claimed in claim 13, or a product of the method of claim 14 or 15, for use in the treatment of animals including humans.

**Patentansprüche**

1. Spiroverbindung der Formel

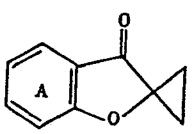

(I)

in der Ring A für einen Benzolring oder einen Naphthalinring steht, wobei der Ring unsubstituiert oder durch mindestens eine der nachstehenden Gruppen substituiert sein kann: $C_{1-6}$-Alkyl, Nitro, Halogen, Amino, das gegebenenfalls selbst substituiert sein kann, Hydroxyl, das gegebenenfalls selbst substituiert sein kann, Acyl und Sulfamoyl.

2. Verbindung nach Anspruch 1, dadurch gekennzeichnet, daß der Ring A durch mindestens eine der nachstehenden Gruppen substituiert ist: Amino, Mono- oder Dialkylamino und Cycloamino.

3. Verbindung nach Anspruch 2, dadurch gekennzeichnet, daß sie in Form eines pharmazeutisch unbedenklichen Säureadditionssalzes vorliegt.

4. Verbindung nach Anspruch 1, dadurch gekennzeichnet, daß der Ring A Benzol ist und der Substituent des Benzolrings Di-$C_{1-4}$-alkylamino oder $C_{2-6}$-Alkanoyl ist.

5. Verbindung nach Anspruch 4, dadurch gekennzeichnet, daß die substituierte Stellung die 5-Stellung des Benzolrings ist.

6. Spiro[benzo[b]furan-2(3H),1'-cyclopropan]3-on.

7. 5-Acetylspiro[benzo[b]furan-2(3H),1'-cyclopropan]3-on.

8. Spiro[naphtho[2,3-b]furan-2(3H),1'-cyclopropan)-3-on.

9. 5-Nitro-spiro[benzo[b]furan-2(3H),1'-cyclopropan]3-on.

10. 5-Amino-spiro[benzo[b]furan-2(3H),1'-cyclopropan]3-on.

11. 5-Methylamino-spiro[benzo[b]furan-2(3H),1'-cyclopropan]3-on.

12. 5-Dimethylamino-spiro[benzo[b]furan-2(3H),1'-cyclopropan]3-on.

13. Pharmazeutische Zusammensetzung enthaltend (A) als Wirkstoff eine wirksame Menge der in Anspruch 1 definierten Spiroverbindung und (B) eine pharmazeutisch unbedenkliches Trägermaterial oder Verdünnungsmittel dafür.

14. Verfahren zur Herstellung einer Spiroverbindung der Formel

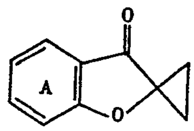

in der der Ring A für einen Benzolring oder einen Naphthalinring steht, wobei der Ring unsubstituiert oder substituiert sein kann durch eine der Gruppen $C_{1-6}$-Alkyl, Nitro, Halogen, Amino, das gegebenenfalls selbst substituiert sein kann, Hydroxyl, das gegebenenfalls selbst substituiert sein kann, Acyl und Sulfamoyl,

dadurch gekennzeichnet, daß eine Verbindung der Formel

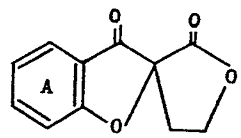

in der Ring A die vorstehend genannte Bedeutung hat, decarboxyliert wird und gegebenenfalls die Verbindung (I) in ein Säureadditionssalz überführt wird, wenn mindestens ein Amino-Substituent am Ring A vorhanden ist, oder in ein Alkalimetallsalz überführt wird, wenn mindestens eine Hydroxyl-Gruppe am Ring A vorhanden ist.

15. Verfahren zur Herstellung einer Spiroverbindung der Formel

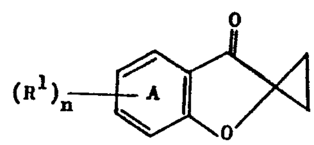

(Ic)

in der Ring A die vorstehend genannte Bedeutung hat und $R^1$ Mono- oder Dialkylamino und n 1 oder 2 ist,

dadurch gekennzeichnet, daß

(1) eine Verbindung der Formel

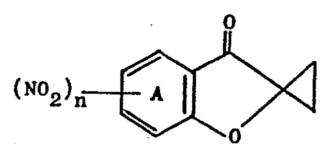

(Ia)

in der n die vorstehend genannte Bedeutung hat, einer Reduktion unterworfen wird und danach die erhaltene Verbindung der Formel

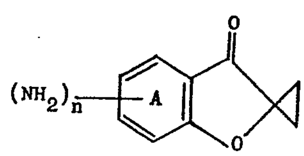

(Ib)

in der n die vorstehend genannte Bedeutung hat, einer reduktiven Alkylierung oder eine Alkylierung mit einem Alkylhalogenid unterworfen wird; oder

(2) eine Verbindung der Formel (Ia) der reduktiven Alkylierung unterworfen wird und, gegebenenfalls, die Verbindung (Ic) in eines ihrer Säureadditionssalze überführt wird.

16. Verbindung (I) oder deren Säureadditionssalze nach Anspruch 1 bis 12 oder eine pharmazeutische Zusammensetzung nach Anspruch 13 oder ein Produkt der Verfahren nach Anspruch 14 oder 15 zur Verwendung für die Behandlung von Tieren und Menschen.

**Revendications**

1. Composé spiro de formule

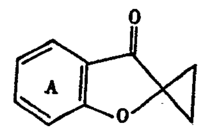

où le noyau A représente un noyau benzène ou un noyau naphtalène, le noyau étant non substitué ou substitué par au moins un alkyle en $C_{1-6}$, nitro, halogène, amino, qui peuvent être substitués, hydroxyle, qui peut être substitué, acyle et sulfamoyle.

2. Composé selon la revendication 1, dans lequel le noyau A est substitué par au moins un amino, mono- ou dialkylamino et cycloamino.

3. Composé selon la revendication 2, sous la forme d'un sel d'addition avec un acide, pharmaceutiquement acceptable.

4. Composé selon la revendication 1, dans lequel le noyau A est le benzène et le substituant du noyau benzénique est un dialkyle en $C_{1-4}$-amino ou alcanoyle en $C_{2-6}$.

5. Composé selon la revendication 4, dans lequel la position substituée est la position 5 du noyau benzène.

6. Spiro-[benzo[b]furan-2(3H),1'-cyclopropane]-3-one.

7. 5-acétylspiro[benzo[b]furane-2(3H),1'-cyclopropane]-3-one.

8. Spiro-[naphto[2,3-b]furan-2(3H),1'-cyclopropane]-3-one.

9. 5-nitro-spiro[benzo[b]furan-2(3H),1'-cyclopropane]-3-one.

10. 5-amino-spiro[benzo[b]furan-2(3H),1'-cyclopropane]-3-one.

11. 5-méthyl-aminospiro[benzo[b]furan-2(3H),1'-cyclopropane]-3-one.

12. 5-diméthylaminospiro[benzo[b]furan-2(3H),1'-cyclopropane]-3-one.

13. Composition pharmaceutique comprenant (A) comme constituant actif, une quantité efficace d'un composé spiro défini dans la revendication 1, et (B) un support ou un diluant pharmaceutiquement acceptable de ce composé.

14. Procédé de préparation d'un composé spiro de formule

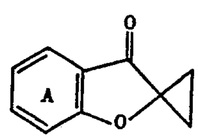

où le noyau A représente un noyau benzène ou un noyau naphtalène, le noyau ètant non substitué ou substitué par au moins an alkyle en $C_{1-6}$, nitro, halgène, amino, qui peut éventuellement être lui-même substitué, hydroxyle qui peut éventuellement être substitué, acyle et sulfamoyle,

ce procédé comprenant la décarboxylation d'un composé de formule

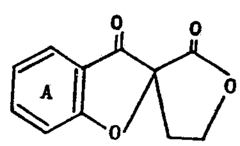

où le noyau A est défini ci-dessus;

et éventuellement la transformation du composé (I) en un sel d'addition avec un acide lorsqu'au moins un substituant amino est présent sur le noyau A et à un sel de métal alcalin lorsqu'au moins un groupe hydroxyle est présent sur le noyau A.

15. Procédé de préparation d'un composé spiro de formule

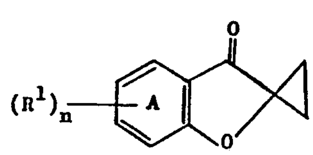

(Ic)

où le noyau A est défini ci-dessus et $R^1$ désigne un mono- ou di-alkylamino et *n* est 1 ou 2, ce procédé comprenant:

(1) la reduction d'un composé de formule

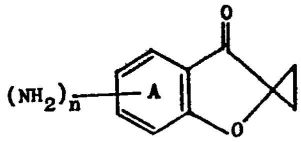

(Ia)

où *n* a la signification ci-dessus indiquée,
et l'alkylation ou l'alkylation réductrice du composé résultant de formule

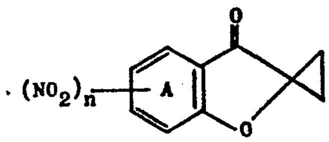

(Ib)

où *n* a la signification ci-dessus indiquée,
avec un halogénure d'alkyle; ou
(2) l'alkylation réductrice d'un composé de formule (Ia); et la transformation éventuelle du composé (Ic) en un sel d'addition avec un acide.

16. Composé (I) ou ses sels d'addition avec un acide, selon les revendications 1—12, ou une composition pharmaceutique selon la revendication 13, ou un produit du procédé selon les revendications 14 ou 15 pour le traitement des animaux y compris les humains.